(19) Europäisches Patentamt
European Patent Office
Office européen des brevets

(11) Numéro de publication : **0 493 273 B1**

(12) # FASCICULE DE BREVET EUROPEEN

(45) Date de publication du fascicule du brevet : **01.06.94 Bulletin 94/22**

(51) Int. Cl.$^5$ : **C07C 51/14**

(21) Numéro de dépôt : **91420452.4**

(22) Date de dépôt : **16.12.91**

(54) **Procédé de préparation de l'acide adipique par hydrocarboxylation d'acides penténiques.**

(30) Priorité : **20.12.90 FR 9016387**

(43) Date de publication de la demande : **01.07.92 Bulletin 92/27**

(45) Mention de la délivrance du brevet : **01.06.94 Bulletin 94/22**

(84) Etats contractants désignés : **BE DE ES FR GB IT NL**

(56) Documents cités :
**EP-A- 0 274 076**
**US-A- 3 579 551**
**US-A- 3 579 552**

(73) Titulaire : **RHONE-POULENC CHIMIE**
**25, quai Paul Doumer**
**F-92408 Courbevoie Cédex (FR)**

(72) Inventeur : **Grosselin, Jean-Michel**
**12, Allée des Erables**
**F-69340 Francheville (FR)**
Inventeur : **Denis, Philippe**
**16, Allée des Troenes**
**F-69150 Decines (FR)**
Inventeur : **Metz, François**
**6, rue de la salle des fêtes**
**F-69390 Vernaison (FR)**
Inventeur : **Delis, Philippe**
**14, Chemin des Rivières**
**F-69370 St-Didier-au-Mont-d'Or (FR)**

(74) Mandataire : **Vignally, Noel et al**
**Rhône-Poulenc Chimie**
**Direction de la Propriété Industrielle**
**Centre de Recherches des Carrières**
**B.P. 62**
**F-69192 Saint-Fons Cédex (FR)**

**EP 0 493 273 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne un procédé de préparation de l'acide adipique par hydrocarboxylation d'acides penténiques, c'est-à-dire par réaction de l'eau et de l'oxyde de carbone sur au moins un acide penténique.

Dans la demande de brevet européen n° 188 209 il est proposé un procédé de préparation d'acides dicarboxyliques linéaires, en particulier de l'acide adipique par réaction d'acides monocarboxyliques insaturés, en particulier l'acide pentène-3 oïque, de l'oxyde de carbone et d'eau en présence d'un catalyseur à base de rhodium et d'un promoteur iodé la réaction étant conduite dans un solvant tel le chlorure de méthylène à une température de 100 à 240°C et sous une pression totale comprise entre 14 et 240 atm ; une température comprise entre 150 et 180°C et une pression totale comprise entre 24 et 40 atmosphères sont estimées préférables.

Le choix du solvant et la teneur en eau du milieu réactionnel sont critiques dans le cadre du procédé en cause ce qui constitue des contraintes difficilement acceptables à l'échelle industrielle.

Le brevet américain n° 3 579 552 enseigne l'utilisation d'un catalyseur à base de rhodium et d'un promoteur iodé dans les réactions d'hydrocarboxylation des oléfines et de certains de leurs dérivés. Toutefois la proportion d'acide linéaire reste insuffisante.

Le brevet américain n° 3 579 551 enseigne l'utilisation d'un catalyseur à base d'iridium et d'un promoteur iode dans les réactions d'hydrocarboxylation des oléfines et de certains de leurs dérivés. Toutefois l'activité d'un tel système reste insuffisante.

Dans un tel contexte il serait manifestement souhaitable de pouvoir disposer d'un système catalytique offrant simultanément une activité et une sélectivité en acide adipique améliorées et permettant de s'affranchir au moins partiellement des contraintes évoquées ci-avant.

La présente invention a donc pour objet un procédé de préparation d'acide adipique par hydrocarboxylation d'au moins un acide penténique en présence de rhodium ou d'un composé du rhodium et d'un promoteur iodé caractérisé en ce que la réaction est conduite également en présence d'au moins un co-catalyseur choisi dans le groupe constitué par l'iridium, le ruthénium, l'osmium et leurs composés.

Par acide penténique, on entend dans le cadre de la présente invention l'acide pentène-2 oïque, l'acide pentène-3 oïque, l'acide pentène-4 oïque et leurs mélanges.

L'acide pentène-4 oïque conduit à de bons résultats, mais reste peu disponible.

L'acide pentène-3 oïque pris isolément ou en mélange avec ses isomères est plus particulièrement approprié compte-tenu de son accessibilité et des résultats satisfaisants auxquels il conduit dans le cadre du présent procédé.

Le procédé selon la présente invention nécessite la présence d'un catalyseur à base de rhodium. N'importe quelle source de rhodium est susceptible d'être mise en oeuvre.

A titre d'exemples de sources de rhodium susceptibles de convenir à la mise en oeuvre du présent procédé on peut citer :

Rh métal ; $Rh_2O_3$ ;
$RhCl_3$ ; $RhCl_3$, $3H_2O$ ;
$RhBr_3$ ; $RhBr_3$, $3H_2O$ ;
$RhI_3$ ; $Rh(NO_3)_3$ ; $Rh(NO_3)_3$, $2H_2O$ ;
$Rh_2(CO)_4Cl_2$ ; $Rh_2(CO)_4Br_2$ ; $Rh_2(CO)_4I_2$ ;
$Rh(CO)Cl[P(C_6H_5)_3]_2$
$Rh[P(C_6H_5)_3]_2(CO)I$
$Rh[P(C_6H_5)_3]_3Br$
$Rh_4(CO)_{12}$ ; $Rh_6(CO)_{16}$ ; $Rh(CO)_2$ (acac) ;
$Rh(Cod)(acac)_2$ ; $Rh(acac)_3$ ;
$Rh_2(Cod)_2Cl_2$ . $Rh_2(CO_2CH_3)_4$ ;
$HRh(CO)[P(C_6H_5)_3]_3$ ;
(Cod = cyclooctadiène-1,5 ; acac : acétylacétonate)

Conviennent plus particulièrement à la mise en oeuvre du présent procédé :

$Rh_2(Cod)_2Cl_2$ ;
$Rh_2(CO)_4Cl_2$ ;
$RhI_3$ ; $RhCl_3$, $3H_2O$ ; $Rh(acac)_3$ ;
$Rh(Cod)(acac)_2$ ; $Rh_2(CO_2CH_3)_4$ ; $Rh_4(CO)_{12}$ ;
et $Rh_{6(CO)16}$.

La quantité de rhodium à mettre en oeuvre peut varier dans de larges limites.

En général, une quantité exprimée en mole de rhodium métallique par litre de milieu réactionnel comprise

entre $10^{-3}$ et $10^{-1}$ conduit à des résultats satisfaisants. Des quantités inférieures peuvent être mises en oeuvre ; on observe toutefois que la vitesse de réaction est faible. Des quantités supérieures n'ont d'inconvénients qu'au plan économique.

De préférence, la concentration de rhodium est comprise entre $5.10^{-3}$ et $10^{-2}$ (inclus) mol/l.

Par promoteur iodé on entend dans le cadre du présent procédé HI et les composés organoiodés capables de générer HI dans les conditions réactionnelles et, en particulier les iodures d'alkyles en $C_1$-$C_{10}$, l'iodure de méthyle étant plus particulièrement préconisé.

On remarquera que des composés tels $RhI_3$ peuvent être à la fois une source utile de rhodium et constituer tout ou partie du promoteur iodé.

La quantité de promoteur iodé à mettre en oeuvre est en général telle que le rapport molaire I/Rh soit supérieur ou égal à 0,1. Il n'est pas souhaitable que ce rapport excède 20. De préférence, le rapport molaire I/Rh est compris entre 1 et 4 (inclus).

La présence d'eau est indispensable à la conduite du procédé selon la présente invention. En général, la quantité d'eau à mettre en oeuvre est telle que le rapport molaire eau/acide(s) penténique(s) soit compris entre 1 et 10 (inclus).

Une quantité inférieure présente l'inconvénient de limiter la conversion. Une quantité supérieure n'est pas souhaitable en raison de la perte d'activité catalytique observée.

Le procédé selon la présente invention nécessite également la présence d'au moins un co-catalyseur choisi dans le groupe constitué par l'iridium, le ruthénium, l'osmium et leur composés.

A titre d'exemple de sources d'iridium susceptibles de convenir à la mise en oeuvre du présent procédé on peut citer :

Ir métallique ; Ir $O_2$ ; $Ir_2$ $O_3$ ;
Ir $Cl_3$ ; Ir $Cl_3$ , 3 $H_2O$ ;
Ir $Br_3$ ; Ir $Br_3$ , $3H_2O$ ;
Ir $I_3$ ;
$Ir_2(CO)_4Cl_2$ ; $Ir_2(CO)_4I_2$ ;
$Ir_2(CO)_8$ ; $Ir_4(CO)_{12}$ ;
$Ir_2(CO)[P(C_6H_5)_3]_2$ I ;
$Ir(CO)[P(C_6H_5)_3]_2$ Cl ;
$Ir[P(C_6H_5)_3]_3I$ ;
$HIr[P(C_6H_5)_3]_3(CO)$ ;
$Ir(acac)(CO)_2$
$[IrCl(Cod]_2$
(Cod : cyclooctadiène-1,5 ; acac : acétylacétonate)

A titre d'exemples de sources de ruthénium susceptibles de convenir à la mise en oeuvre du présent procédé, on peut citer :

Ru métallique ; $RuO_2$ ; $RuO_4$
$RuCl_3$ ; $RuCl_3$, $3H_3O$ ; $RuBr_3$ ; $RuI_3$ ; $Ru(OAc)_3$ ; $RuCl_4$
$Ru(CO)_5$ ; $Ru(CO)_{12}$ ; $[Ru(CO)_3Br_2]_2$ ; $Ru(CO)_4I_2$
$Ru(acac)_3$ ; Ru $Cl(\eta$-$C_3H_5)$

A titre d'exemple de sources d'osmium suceptibles de convenir à la mise en oeuvre du présent procédé, on peut citer :

Os métallique ; OsO ; $Os_2O_3$ ; $OsO_2$ : OsOF4 ·
$OsCl_3$ ; $OsBr_3$ ; $OsCl_3$ , $3H_2O$ ;
$OsCl_4$ ;
$Os_3(CO)_{12}$

Parmi les sources d'iridium, conviennent plus particulièrement à la mise en oeuvre du présent procédé :

$IrCl_3$ ; $IrCl(Cod)_2$ ; $Ir_4(CO)_{12}$

Parmi les sources de ruthénium, conviennent plus particulièrement à la mise en oeuvre du présent procédé :

$RuO_2$ , $H_2O$ ;
$RuCl_3$, $H_2O$ ; $RuCl_2(Cod)$ ; $Ru_3(CO)_{12)}$ ; $Ru(acac)_3$

Parmi les sources d'osmium, conviennent plus particulièrement à la mise en oeuvre du présent procédé :

$OsCl_3$ ; $Os_3(CO)_{12}$

Le cocatalyseur est avantageusement choisi parmi l'iridium et ses composés.

Le rapport molaire du cocatalyseur exprimé en métal M par rapport au rhodium peut varier dans de larges limites par exemple entre 0,05 et 50 inclus.

De préférence le rapport M/Rh est compris entre 0,1 et 5 inclus.

Le procédé selon la présente invention est avantageusement conduit en phase liquide. Bien entendu, le milieu réactionnel peut renfermer un solvant ou diluant. A titre d'exemples de solvants ou diluants susceptibles d'être mis en oeuvre dans le cadre du présent procédé on peut citer : les acides carboxyliques aliphatiques saturés ou aromatiques, comportant un maximum 20 atomes de carbone, et les hydrocarbures aliphatiques saturés ou aromatiques, chlorés ou non, liquides dans les conditions réactionnelles.

Selon une variante avantageuse du présent procédé on recourt à un acide carboxylique aliphatique saturé en $C_1$-$C_6$ et, de préférence, à l'acide acétique.

Selon une autre variante avantageuse du présent procédé on recourt à un hydrocarbure aliphatique saturé chloré et, de préférence au chlorure de méthylène.

La quantité de solvant ou de diluant, lorsqu'un tel solvant ou diluant est présent dans le milieu réactionnel peut atteindre 99 % en volume dudit milieu. Pour une bonne mise en oeuvre de l'invention cette quantité sera supérieure ou égale à 10 % et, de préférence comprise entre 30 et 90 % en volume (inclus).

La température de réaction est généralement comprise entre 100 et 240°C. Pour une bonne mise en oeuvre de la présente invention la température sest comprise entre 160 et 190°C inclus.

La réaction est conduite sous pression supérieure à la pression atmosphérique. La pression totale peut varier dans de larges limites par exemple entre 2 et 150 bar. En général, de bons résultats sont observés pour des pressions comprises entre 5 et 50 bar.

On peut utiliser de l'oxyde de carbone sensiblement pur ou de qualité technique, tel qu'il se présente dans le commerce.

En fin de réaction ou du temps imparti à celle-ci on sépare l'acide adipique par tout moyen approprié, par exemple par extraction et/ou cristallisation.

Les exemples ci-après illustrent la présente invention.

## EXEMPLES

### EXEMPLE 1 :

Dans une ampoule de verre on introduit successivement 0,1 mmol de rhodium sous forme de $[RhCl(Cod)]_2$, 0,1 mmol d'iridium sous forme de $IrCl_3$, 20 mmol d'acide pentène-3 oïque, 35 mmol d'eau, 0,57 mmol d'HI sous forme d'une solution aqueuse à 57 % en poids et 10 ml d'acide acétique.

L'ampoule est alors placée dans un autoclave en acier inoxydable (HASTELLOY B2) et de 125 cm3 de capacité, préalablement purgé à l'argon.

L'autoclave est fermé hermétiquement, placé dans un four agité et raccordé à l'alimentation de gaz sous pression.

On admet à froid 2 bar de C0 dans l'autoclave et on le porte à 175°C en 25 minutes. La pression dans l'autoclave est alors de 4 bar. on régule celle-ci à 10 bar.

Après 30 minutes de réaction en température, l'absorption de C0 a cessé ; l'autoclave est alors refroidi et dégazé.

La masse réactionnelle est analysée par chromatographie en phase gazeuse et par chromatographie liquide haute performance.

Les quantités de produits formés (rendement molaire par rapport à l'acide pentène-3 oïque charge) sont les suivantes :

acide valérique (Pa) : 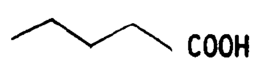 < 1 %

acide pentène-2 oïque (P2) : 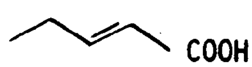 = 6 %

méthyl-4 butyrolactone (M4L) : 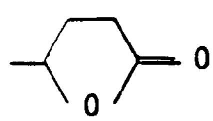 = 18 %

acide éthylsuccinique (A3) : 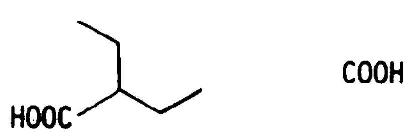 = 5,7 %

acide méthylglutarique (A2) : 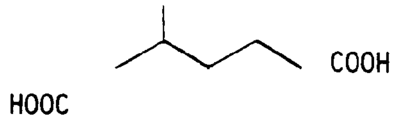 = 14,7 %

acide adipique (A1) : 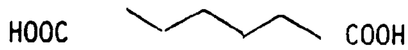 = 48 %

Le taux de linéarité (L) est de 76 %
Le taux de transformation de l'acide pentène-3 oïque (TT) est de 100 %.

EXEMPLES 2 et 3 ; Essais Témoins (a) et (b) :

Dans l'autoclave et selon le mode opératoire, décrits pour l'exemple 1, on réalise une première série d'essais, en ne modifiant que la nature du co-catalyseur.

Les conditions particulières ainsi que les résultats obtenus sont rassemblés dans le tableau I ci-après :

TABLEAU I

| Réf. | Co-catalyseur | TT % | RT (%) | | L (%) |
|---|---|---|---|---|---|
| | | | M4L | A1 | |
| 1 | IrCl3 | 100 | 18 | 48 | 76 |
| 2 | RuCl3 | 97 | 40 | 38 | 65 |
| 3 | OsCl3 | 95 | 38 | 32 | 64 |
| a | - | 100 | 42 | 26 | 55 |
| b(*) | IrCl3 | 83 | 86 | 1,2 | ε |

(*) essai réalisé en l'absence de rhodium ; durée de réaction 120 mn.

EXEMPLES 4 à 8 :

Dans l'autoclave et selon le mode opératoire, décrits dans l'exemple 1 on réalise une seconde série d'essais sur une charge renfermant à chaque fois divers composes du ruthénium comme co-catalyseur dont la nature figure au tableau II ci-après. Les résultats mentionnés dans ce tableau sont obtenus toutes conditions égales par ailleurs et t y représente la durée de réaction.

TABLEAU II

| Réf. | Co-catalyseur | t mn | TT (%) | RT(%) | | L (%) |
|---|---|---|---|---|---|---|
| | | | | M4L | A1 | |
| 4 | RuCl3,H2O | 60 | 89 | 23 | 45 | 66 |
| 5 | RuCl2(Cod) | 300 | 93 | 29 | 44 | 70 |
| 6 | Ru3(CO)12 | 90 | 70 | 24 | 47 | 70 |
| 7 | Ru(acac)3 | 60 | 67 | 25 | 42 | 66 |
| 8 | RuO2,H2O | 90 | 81 | 21 | 44 | 62 |

EXEMPLE 9 :

Dans l'autoclave et selon le mode opératoire, décrits pour l'exemple 1, on réalise un essai sur une charge renfermant 0,1 mmol de rhodium sous forme de $RhI_3$, 0,1 mmol d'iridium sous forme de $IrCl_3$, 20 mmol d'acide pentène-3 oïque, 35 mmol d'eau et 10 mmol d'acide acétique.

Après 35 minutes de réaction à 175°C sous 10 bar de pression totale, les résultats obtenus sont les suivants :

TT (%) = 100
AI (%) = 64
M4L (%) = 14
L (%) = 86

EXEMPLES 10 à 17 :

On réalise une troisième série d'essais sur une charge identique à celle utilisée à l'exemple 9 en modifiant la température de réaction (T) et/ou la pression totale en température (P). Les conditions particulières ainsi que les résultats obtenus sont rassemblés dans le tableau III ci-après :

TABLEAU III

| Réf. | T °C | P (bar) | t (mn) | TT % | RT(%) | | L % |
|---|---|---|---|---|---|---|---|
| | | | | | M4L | Al | |
| 10 | 175 | 5 | 45 | 100 | 10 | 67 | 82 |
| 11 | " | 8 | 35 | 100 | 9 | 67 | 81 |
| 9 | " | 10 | 35 | 100 | 14 | 64 | 86 |
| 12 | " | 25 | 30 | 100 | 10 | 63 | 76 |
| 13 | " | 47 | 30 | 99 | 10 | 66 | 80 |
| 14 | " | 100 | 60 | 83 | 10 | 53 | 65 |
| 15 | 190 | 15 | 30 | 100 | 10 | 65 | 78 |
| 16 | 160 | 10 | 65 | 99 | 9 | 65 | 79 |
| 17 | 140 | 10 | 105 | 59 | 6 | 48 | 58 |

EXEMPLE 18 :

On reproduit l'exemple 9 ci-avant en remplaçant $IrCl_3$ par la même quantité de $[IrCl(Cod)]_2$.

Toutes conditions égales par ailleurs, on obtient sensiblement les mêmes résultats.

EXEMPLE 19 :

On reproduit l'exemple 9 ci-avant en remplaçant $IrCl_3$ par la même quantité de $Ir_4(CO)_{12}$.

Toutes conditions égales par ailleurs, on obtient sensiblement les mêmes résultats.

EXEMPLE 20 :

On reproduit l'exemple 9 ci-avant en remplaçant $RhI_3$ par une quantité équivalente de $[RhCl(Cod)]_2$ et en ajoutant 0,3 mmol d'HI à la charge.

Toutes conditions égales par ailleurs, on obtient sensiblement les mêmes résultats.

EXEMPLES 21 à 26 ; essai témoin c :

On réalise une quatrième série d'essais sur une charge analogue à celle utilisée à l'exemple 9 à ceci près que la quantité de $IrCl_3$ et/ou la nature de l'acide penténique chargé diffère. Les conditions particulières ainsi que les résultats obtenus à 175°C sous une pression totale de 10 bar sont rassemblés dans le tableau IV ci-

après :

TABLEAU IV

| Réf. | Ir/Rh | Acide penténique | t(mn) | TT (%) | RT(%) | | L (%) |
|---|---|---|---|---|---|---|---|
| | | | | | M4L | Al | |
| 21 | 0,5 | pentène-3 oïque | 90 | 100 | 17 | 58 | 76 |
| 22 | 1 | " | 60 | 100 | 12 | 62 | 83 |
| 23 | 2 | " | 50 | 100 | 17 | 54,5 | 85 |
| 24 | 5 | " | 50 | 100 | 39 | 28 | 90 |
| 25 | 1 | pentène-4 oïque | 30 | 100 | 10 | 69 | 81 |
| 26 | 1 | pentène-2 oïque | 75 | 92 | 12,5 | 50 | 82 |
| c | 0 | pentène-2 oïque | 120 | 8 | 11 | 23 | 45 |

EXEMPLES 27 à 29 :

On réalise une cinquième série d'essais sur une charge analogue à celle de l'exemple 9 en faisant varier la concentration en eau du milieu réactionnel tout en conservant le volume total (eau + acide acétique) constant et égal à 12,5 ml.

Les conditions particulières ainsi que les résultats obtenus sont rassemblés dans le tableau V ci-après :

TABLEAU V

| Réf. | $H_2O$(g/l) | t (mn) | TT (%) | RT(%) | | L % |
|---|---|---|---|---|---|---|
| | | | | M4L | Al | |
| 27 | 29 | 40 | 100 | 12 | 67 | 81 |
| 9 | 47 | 35 | 100 | 14 | 64 | 86 |
| 28 | 147 | 50 | 90 | 19 | 48 | 65 |
| 29 | 290 | 70 | 61 | 19 | 27 | 46 |

EXEMPLE 30 à 32 :

Dans l'autoclave et selon le mode opératoire, décrits pour l'exemple 1, on réalise une sixième série d'essais sur une charge renfermant 0,1 mmol d'iridium sous forme de $IrCl_3$, 20 mmol d'acide pentène-3 oïque, 39 mmol d'eau, 10 ml de solvant dont la nature précise figure au tableau VI ci-après, 0,1 mmol de rhodium sous forme de $[RhCl(Cod)]_2$ (exemples 30 et 31) ou sous forme de $RhI_3$ (exemple 32). Dans les exemples 30 et 31 la charge renferme en outre 0,3 mmol d'HI. Les résultats obtenus à 175°C ainsi que les conditions particulières sont rassemblés dans le tableau VI ci-après :

TABLEAU VI

| Réf. | Nature du solvant | P (bar) | t (mn) | TT (%) | RT(%) | | L % |
|---|---|---|---|---|---|---|---|
| | | | | | M4L | A1 | |
| 30 | Toluène | 10 | 60 | 70 | 6 | 21 | 75 |
| 31 | Chlorure de méthylène | 30 | 60 | 81 | 6 | 50 | 75 |
| 32 | Acide Valérique | 28 | 100 | 95 | 7 | 56 | 70 |

## Revendications

1. Procédé de préparation de l'acide adipique par hydrocarboxylation d'au moins un acide penténique en présence de rhodium ou d'un composé du rhodium et d'un promoteur iodé caractérisé en ce que la réaction est également conduite en présence d'au moins un co-catalyseur choisi dans le groupe constitué par l'iridium, le ruthénium, l'osmium et leurs composés.

2. Procédé selon la revendication 1, caractérisé en ce que le rapport molaire du co-catalyseur exprimé en métal M par rapport au rhodium est compris entre 0,05 et 50 inclus.

3. Procédé selon la revendication 1 ou 2, caractérisé en ce que le co-catalyseur est choisi parmi l'iridium et ses composés.

4. Procédé selon l'une quelconque des revendications précédentes, caractérisé en ce que la concentration en rhodium est comprise entre $10^{-3}$ et $10^{-1}$ mol/l.

5. Procédé selon l'une quelconque des revendications précédentes, caractérisé en ce que le rapport molaire I/Rh est supérieur ou égal à 0,1 et, de préférence inférieur ou égal à 20.

6. Procédé selon l'une quelconque des revendications précédentes, caractérisé en ce que le rapport molaire eau/acide(s) penténique(s) est compris entre 1 et 10.

7. Procédé selon l'une quelconque des revendications précédentes, caractérisé en ce que la réaction est conduite en présence d'un acide carboxylique sature en $C_1$-$C_6$.

8. Procédé selon la revendication 7 caractérisé en ce que l'acide carboxylique saturé est l'acide acétique.

9. Procédé selon l'une quelconque des revendications 1 à 6, caractérisé en ce que la réaction est conduite en présence de chlorure de méthylène.

10. Procédé selon l'une quelconque des revendications précédentes, caractérisé en ce que le rapport M/Rh est compris entre 0,1 et 5 inclus.

11. Procédé selon l'une quelconque des revendications précédentes, caractérisé en ce que le rapport I/Rh est compris entre 1 et 4 inclus.

12. Procédé selon l'une quelconque des revendications précédentes, caractérisé en ce que la température de réaction est comprise entre 100 et 240°C et, de préférence, entre 160 et 190°C, inclus.

13. Procédé selon l'une quelconque des revendications précédentes, caractérisé en ce que la pression est comprise entre 2 et 150 bar et, de préférence entre 5 et 50 bar inclus.

## Claims

1. Process for preparing adipic acid by hydrocarboxylation of at least one pentenic acid in the presence of rhodium or a rhodium compound and an iodinated promoter, characterized in that the reaction is also performed in the presence of at least one cocatalyst chosen from the group consisting of iridium, ruthenium, osmium and their compounds.

2. Process according to claim 1, characterized in that the mole ratio of the cocatalyst expressed as metal M relative to the rhodium is between 0.05 and 50 inclusive.

3. Process according to claim 1 or 2, characterized in that the cocatalyst is chosen from iridium and its compounds.

4. Process according to any one of the preceding claims, characterized in that the rhodium concentration is between $10^{-3}$ and $10^{-1}$ mol/l.

5. Process according to any one of the preceding claims, characterized in that the I/Rh mole ratio is greater than or equal to 0.1, and preferably less than or equal to 20.

6. Process according to any one of the preceding claims, characterized in that the water/pentenic acid(s) mole ratio is between 1 and 10.

7. Process according to any one of the preceding claims, characterized in that the reaction is performed in the presence of a saturated $C_1$-$C_6$ carboxylic acid.

8. Process according to claim 7, characterized in that the saturated carboxylic acid is acetic acid.

9. Process according to any one of claims 1 to 6, characterized in that the reaction is performed in the presence of methylene chloride.

10. Process according to any one of the preceding claims, characterized in that the N/Rh ratio is between 0.1 and 5 inclusive.

11. Process according to any one of the preceding claims, characterized in that the I/Rh ratio is between 1 and 4 inclusive.

12. Process according to any one of the preceding claims, characterized in that the reaction temperature is between 100 and 240°C, and preferably between 160 and 190°C, inclusive.

13. Process according to any one of the preceding claims, characterized in that the pressure is between 2 and 150 bar, and preferably between 5 and 50 bar, inclusive.

## Patentansprüche

1. Verfahren zur Herstellung von Adipinsäure durch Hydrocarboxylierung mindestens einer Pentensäure in Gegenwart von Rhodium oder einer Rhodiumverbindung und eines iodierten Promotors, dadurch gekennzeichnet, daß die Reaktion auch in Gegenwart mindestens eines Cokatalysators durchgeführt werden kann, ausgewählt aus der aus dem Iridium, dem Ruthenium, dem Osmium und ihren Verbindungen bestehenden Gruppe.

2. Verfahren nach Anspruch 1, dadurch gekennzeichnet, daß das molare Verhältnis des Cokatalysators, ausgedrückt als Metall M, in Bezug auf das Rhodium zwischen 0,05 und 50 einschließlich liegt.

3. Verfahren nach Anspruch 1 oder 2, dadurch gekennzeichnet, daß der Cokatalysator aus Iridium und seinen Verbindungen ausgewählt ist.

4. Verfahren nach einem beliebigen der vorhergehenden Ansprüche, dadurch gekennzeichnet, daß die Konzentration an Rhodium zwischen $10^{-3}$ und $10^{-1}$ mol/l liegt.

5. Verfahren nach einem beliebigen der vorhergehenden Ansprüche, dadurch gekennzeichnet, daß das molare Verhältnis I/Rh größer als oder gleich 0,1 und vorzugsweise kleiner als oder gleich 20 ist.

6. Verfahren nach einem beliebigen der vorhergehenden Ansprüche, dadurch gekennzeichnet, daß das molare Verhältnis Wasser/Pentensäure(n) zwischen 1 und 10 liegt.

7. Verfahren nach einem beliebigen der vorhergehenden Ansprüche, dadurch gekennzeichnet, daß die Reaktion in Gegenwart einer gesättigten Carbonsäure mit $C_1$-$C_6$ durchgeführt wird.

8. Verfahren nach Anspruch 7, dadurch gekennzeichnet, daß die gesättigte Carbonsäure Essigsäure ist.

9. Verfahren nach einem beliebigen der vorhergehenden Ansprüche 1 bis 6, dadurch gekennzeichnet, daß die Reaktion in Gegenwart von Methylenchlorid durchgeführt wird.

10. Verfahren nach einem beliebigen der vorhergehenden Ansprüche, dadurch gekennzeichnet, daß das Verhältnis M/Rh zwischen 0,1 und 5 einschließlich liegt.

11. Verfahren nach einem beliebigen der vorhergehenden Ansprüche, dadurch gekennzeichnet, daß das Verhältnis I/Rh zwischen 1 und 4 einschließlich liegt.

12. Verfahren nach einem beliebigen der vorhergehenden Ansprüche, dadurch gekennzeichnet, daß die Reaktionstemperatur zwischen 100 und 240°C und vorzugsweise zwischen 160 und 190°C einschließlich liegt.

13. Verfahren nach einem beliebigen der vorhergehenden Ansprüche, dadurch gekennzeichnet, daß der Druck zwischen 2 und 150 bar und vorzugsweise zwischen 5 und 50 bar einschließlich liegt.